# EUROPEAN PATENT APPLICATION

(11) **EP 2 420 276 A2**
(43) Date of publication of application: **22.02.2012**
(21) Application number: 10764590.5
(22) Date of filing: 26.03.2010
(51) Int. Cl.: A61M 5/175, A61M 5/168

(54) **DRUG FILLING AND FLOW CONTROL APPARATUS, AND DRUG INJECTION APPARATUS COMPRISING SAME**

(30) Priority: 13.04.2009 KR 20090031735
(71) Applicant: E-WHA Fresenius Kabi Inc., Gyeonggi-do 435-831 (KR)
(72) Inventor: KIM, Yong-Nyun, Goyang-si Gyeonggi-do 412-220 (KR)
(74) Representative: Peckmann, Ralf
(86) International application number: PCT/KR2010/001881
(87) International publication number: WO 2010/120051

(57) **Abstract**

The present invention relates to a medicine charging and flow controlling device, which charges a medicine injection apparatus with a medicine or controls the flow of the charged medicine, and a medicine injection apparatus having the same. The medicine charging and flow controlling device has a one-way valve 40 installed to a medicine charging port 24 which is charged with an external medicine through a syringe, thereby preventing the medicine from flowing back and blocking the introduction of an external air so that the medicine is not contaminated.

## Description

### [Technical Field]

The present invention relates to a medicine charging and flow controlling device, which charges a medicine injection apparatus, which injects the medicine into a patient at a consistent flow rate, with a medicine and controls the flow of the charged medicine, and a medicine injection apparatus having the same.

### [Background Art]

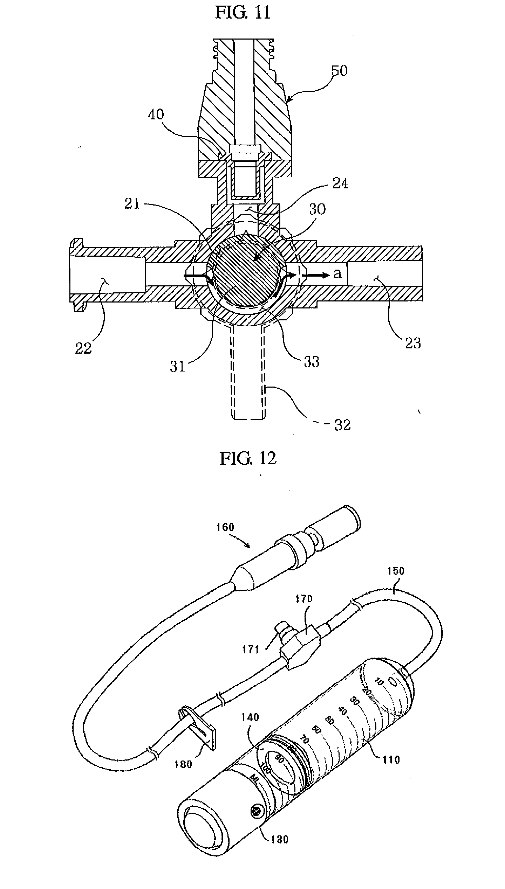

A conventional medicine injection apparatus disclosed in Korean Patent Registration No. 10-0507593 is known in the art. The medicine injection apparatus as shown in FIG. 12 includes a substantially cylindrical cylinder 110, a piston 140 sliding in the cylinder 110, a medicine storage space formed at the front of the piston 140 in the cylinder 110, a gas supply unit 130 disposed at the rear of the piston 140 to push the piston 140 forwards, a medicine moving tube 150 connected to the cylinder 110, and a terminal cap 160 connected to the end of the tube 150. In addition, a medicine supply valve 170 for supplying a medicine through the tube 150 into the cylinder 110 is connected to the tube 150. Unexplained reference numeral 180 designates a clip member.

However, the medicine injection apparatus as described above has the following problems. In other words, the medicine supply valve 170 provided in the conventional medicine injection apparatus does not have the configuration for preventing a medicine supplied from an external medicine supply device such as a syringe through a syringe connection unit 171 from flowing in a direction opposite to the supplying direction. Therefore, when a syringe is connected to the syringe connection unit 171 to charge the cylinder 110 of the medicine injection apparatus with the medicine of the syringe, the medicine previously supplied in the cylinder 110 may be pushed toward the medicine supply valve 170 by an unintended operation of the piston 140 or the pressure of a residual air in the cylinder 110 to thereby flow back to the syringe connection unit 171 of the medicine supply valve 170.

In addition, in case of the medicine supply valve 170 provided in the medicine injection apparatus, in the processes where the syringe is removed from the syringe connection unit 171 to close the medicine supply valve 170 after the medicine is completely charged and the flow direction of the medicine is changed, the medicine charged in the cylinder 110 may leak through the syringe connection unit 171 of the medicine supply valve 170. The leakage of the medicine may contaminate the medicine or waste the expensive medicine.

### [Disclosure]

### [Technical Problem]

Therefore, the present invention is conceived to solve the aforementioned problems in the conventional medicine injection apparatus, particularly the medicine supply valve. An object of the present invention is to provide a medicine charging and flow controlling device for preventing a medicine from leaking or flowing back through a medicine supply valve when charging a medicine injection apparatus with the medicine, by connecting the medicine supply valve to a syringe or the like, and a medicine injection apparatus having such a medicine charging and flow controlling device.

In addition, another object of the present invention is to provide a medicine charging and flow controlling device for preventing a medicine from leaking or flowing back to the medicine injection hole of the medicine supply valve when the medicine supply valve is closed after the medicine is completely charged and the flow direction of the medicine is changed and for preventing the medicine to be lost or contaminated by preventing the penetration of an external air.

### [Technical Solution]

According to an aspect of the present invention for achieving the objects, there is provided a medicine charging and flow controlling device, which includes a valve body having an inlet port communicating with an upstream conduit extending to communicate with a medicine storage space of a medicine injection apparatus, an outlet port communicating with a downstream conduit, and a medicine charging port allowing a medicine being introduced therein from the outside; a flow control unit coupled with the valve body and having a connection passage allowing two of the inlet port, the outlet port and the medicine charging port to selectively communicate with each other; and a one-way valve installed to the medicine charging port of the valve body to allow the medicine to flow only in a direction from an inlet of the medicine charging port to an outlet thereof and to block the flow of the medicine in the opposite direction.

In the medicine charging and flow controlling device according to one embodiment of the present invention, the flow control unit may be rotatably coupled in the valve body and allow two ports among the inlet port, the outlet port and the medicine charging port to selectively communicate with each other according to a rotation position thereof. For example, the flow control unit may be a swivel spool having a rotating handle.

Further, in the medicine charging and flow controlling device according to one embodiment of the present invention, the one-way valve may be made of an elastic material and have an inlet and an outlet, and a cross section of a flowing path of the one-way valve gradually decreases from the inlet to the outlet so that the front end of the outlet is closed by both sidewalls being in close contact with each other by elasticity.

For example, the one-way valve may be configured as a duckbill valve. However, the present invention is not limited thereto, but may use any one-way valve which allows a liquid to flow in one direction and blocks the back flow of the liquid.

Furthermore, in the medicine charging and flow controlling device according to one embodiment of the present invention, the connection passage provided in the flow control unit may be formed on the sidewall of the flow control unit along the circumferential direction, and the connection passage may be formed in a range greater than 180 degrees and not more than 190 degrees with respect to the central axis.

Meanwhile, in the medicine charging and flow controlling device according to one embodiment of the present invention, a connection part for connecting an external medicine supply device for supplying a medicine from the outside may be coupled to the medicine charging port of the valve body. The connection part is preferably a swivel connection part for rotatably connecting the external medicine supply device such as a syringe.

In the medicine charging and flow controlling device according to one embodiment of the present invention, the connection part may include a medicine charging port coupling unit coupled to the medicine charging port, and a rotatable connection unit for rotatably connecting the external medicine supply device. In addition, the medicine charging port coupling unit may be bonded to the medicine charging port by ultrasonic welding to maintain airtightness therebetween. In addition, a sealing member may be inserted and installed between the medicine charging port coupling unit and the medicine charging port.

According to another aspect of the present invention, there is provided a medicine injection apparatus comprising the medicine charging and flow controlling device as described above.

### [Advantageous Effects]

According to the present invention so configured, when a medicine injection apparatus is charged with a medicine from an external medicine supply device such as a syringe, a back flow of the medicine and penetration of an external air may be effectively prevented, so that it is possible to prevent the medicine previously charged in the medicine injection apparatus from being leaked outside or contaminated due to the contact with the external air.

### [Description of Drawings]

The above and other technical subjects and features of the present invention will be more apparent to those having ordinary skill in the art by the following description on the embodiments of the present invention with reference to the accompanying drawings, in which:
FIG. 1 is a schematic view showing a medicine injection apparatus to which a medicine charging and flow controlling device according to an embodiment of the present invention is installed;
FIG. 2 is an exploded perspective view showing the medicine charging and flow controlling device according to the embodiment of the present invention;
FIG. 3 is a sectional view showing the medicine charging and flow controlling device according to the embodiment of the present invention in an assembled state;
FIG. 4 is a perspective view showing a one-way valve of the medicine charging and flow controlling device according to the embodiment of the present invention;
FIG. 5 is a longitudinal sectional view showing the one-way valve of FIG. 4;
FIGS. 6 to 8 show use states of the medicine injection apparatus to which the medicine charging and flow controlling device according to the embodiment of the present invention is installed;
FIGS. 9 to 11 are sectional views showing the medicine charging and flow controlling device according to the embodiment of the present invention, which correspond to the use states of FIGS. 6 to 8, respectively; and
FIG 12 is a schematic view showing a conventional medicine injection apparatus.

### [Best Mode]

Hereinafter, preferred embodiments according to the present invention will be described in detail with reference to the accompanying drawings. The following embodiments of the present invention are just to implement the present invention and are not intended to limit or restrict the scope of the present invention. All techniques easily conceivable by those skilled in the art from the detailed descriptions and embodiments of the present invention are interpreted as belonging to the scope of the present invention. The references cited herein are incorporated herein by reference.

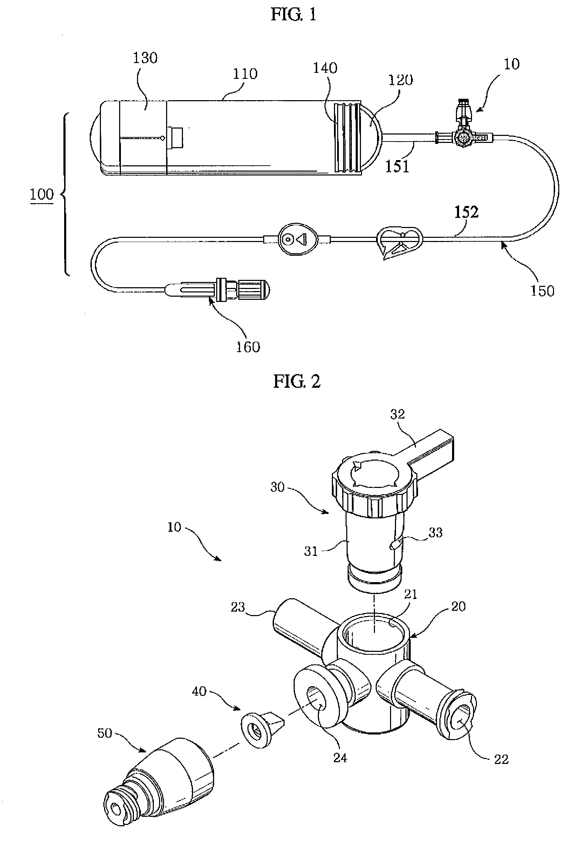

As shown in FIG. 1, a medicine charging and flow controlling device 10 according to an embodiment of the present invention is connected to a medicine moving tube 150 which guides a medicine charged in a medicine storage space 120 in a cylinder 110 of a medicine injection apparatus 100 to be discharged to the outside.

In addition, the medicine injection apparatus 100 includes the cylinder 110 having a cylindrical shape, a piston 140 sliding in the cylinder 110, the medicine storage space 120 formed at the front of the piston 140 in the cylinder 110, a gas supply unit 130 disposed at the rear of the piston 140 to push the piston 140 forwards, the medicine moving tube 150 connected to the cylinder 110, and a terminal cap 160 connected to the end of the tube 150. A syringe needle or catheter is connected to the terminal cap 160.

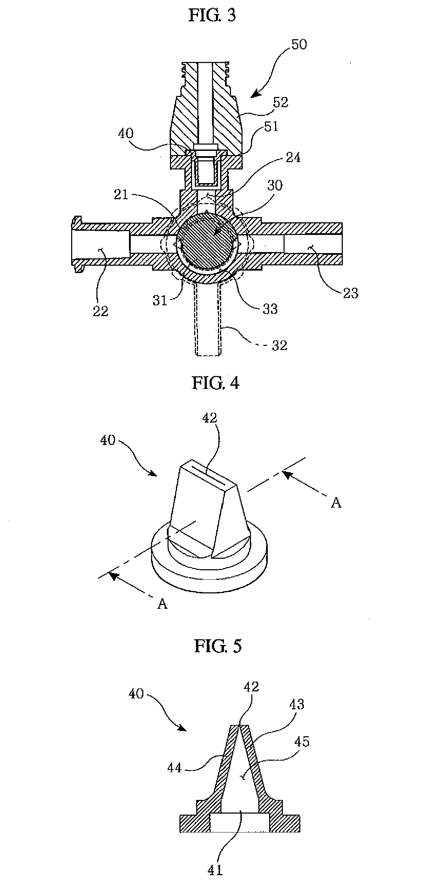

As shown in FIGS. 2 and 3, the medicine charging and flow controlling device 10 according to the embodiment of the present invention is configured to include a valve body 20 connected to the tube 150, a flow control unit 30 for controlling a flow direction of the medicine passing through the valve body 20, a one-way valve 40 for allowing the medicine supplied from an external medicine supply device (not shown) to flow only in a charging direction, and a connection part 50 for connecting an external medicine supply device.

The valve body 20 has a hole 21 in a central axis direction so that the flow control unit 30 may be slidably received in the hole 21. In addition, the valve body 20 includes an inlet port 22 communicating with an upstream conduit 151 extending to communicate with a medicine storage space 120 of the medicine injection apparatus 100, an outlet port 23 connected to a downstream conduit 152, and a medicine charging port 24 to which an external medicine supply device is connected.

The inlet port 22 and the outlet port 23 are provided to oppose to each other along the direction intersecting the hole 21 of the valve body 20. The medicine charging port 24 is provided between the inlet port 22 and the outlet port 23 in a direction of approximately 90 degrees with respect to the inlet and outlet ports. The arrangement of the ports is a preferred example of the present invention, and the present invention is not limited thereto.

The flow control unit 30 may include a body 31 having a substantially cylindrical shape, and a rotating handle 32 provided at the upper end thereof. For example, the flow control unit 30 has a connection passage 33 formed over a certain region along the circumferential direction of an outer sidewall so that two of the inlet port 22, the outlet port 23 and the medicine charging port 24 of the valve body 20 selectively communicate with each other according to the rotational direction of the flow control unit 30.

The connection passage 33 of the flow control unit 30 is preferably formed on the outer sidewall over the region greater than about 180 degrees and not more than 190 degrees along the circumferential direction so that the inlet port 22 and the outlet port 23 of the valve body 20 disposed at an angle of about 180 degrees communicate with each other.

Meanwhile, the one-way valve 40 may be made of a material with high elasticity such as silicone rubber. However, the present invention is not limited thereto but may use one-way valves made of various materials.

In addition, as shown in FIGS. 4 and 5, the one-way valve 40 may include a charging flow path 45 having an inlet 41 and an outlet 42, and the cross section of the charging flow path 45 gradually decreases from the inlet 41 toward the outlet 42 so that the outlet 42 is closed by both sidewalls 43 and 44 which are in close contact with each other by elasticity.

For example, the one-way valve 40 may be configured as a duckbill valve. However, the present invention is not limited thereto, but may use any one-way valve which allows a liquid to flow in one direction and prevents the liquid from flowing in the opposite direction.

The one-way valve 40 is inserted and installed into the front end of the inlet of the medicine charging port 24 of the valve body 20. A connection part 50 for connecting an external medicine supply device such as a syringe is disposed at the upper side of the one-way valve 40 and is coupled with the medicine charging port 24 (see FIGS. 2 and 3).

In other words, as shown in FIGS. 2 and 3, as the connection part 50 is coupled to the medicine charging port 24 of the valve body 20, the one-way valve 40 inserted and installed into the front end of the inlet of the medicine charging port 24 may be fixedly placed between the connection part 50 and the medicine charging port 24.

Meanwhile, the connection part 50 is preferably a swivel connection part which may rotatably connect an external medicine supply device such as a syringe, but the present invention is not limited thereto.

When a medicine is supplied from a medicine supply device such as a syringe connected to the connection part 50 under a certain pressure, both sidewalls 43 and 44 of the charging flow path 45 of the one-way valve 40 elastically expand due to the charging pressure of the medicine and open the outlet 42 of the charging flow path 45. The medicine is introduced and charged through the opened outlet 42 of the charging flow path 45.

On the contrary, in a case where a medicine is not supplied from the medicine supply device or the pressure of the supplied medicine is not greater than the elastically restoring force of the sidewalls 43 and 44 of the charging flow path 45, the outlet 42 of the one-way valve 40 is closed so that the already charged medicine does not flow back toward the medicine supply device.

Meanwhile, the connection part 50 includes a medicine charging port coupling unit 51 coupled to the medicine charging port 24, and a rotatable connection unit 52 for rotatably connecting, for example an external medicine supply device (see FIG. 3). In addition, the medicine charging port coupling unit 51 may be bonded to the medicine charging port 24 by ultrasonic welding or the like to maintain airtightness therebetween. Additionally, a sealing member (not shown) may be inserted and installed between the medicine charging port coupling unit 51 and the medicine charging port 24. In addition, the connection part 50 may maintain its coupling state without distortion even though the medicine supply device connected thereto rotates.

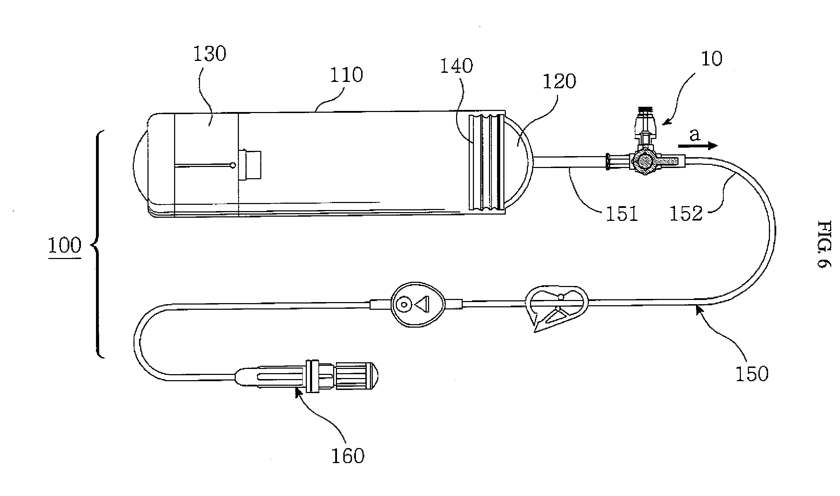

The operation of the medicine charging and flow controlling device 10 according to the embodiment of the present invention so configured will be described with reference to FIGS. 6 to 8.

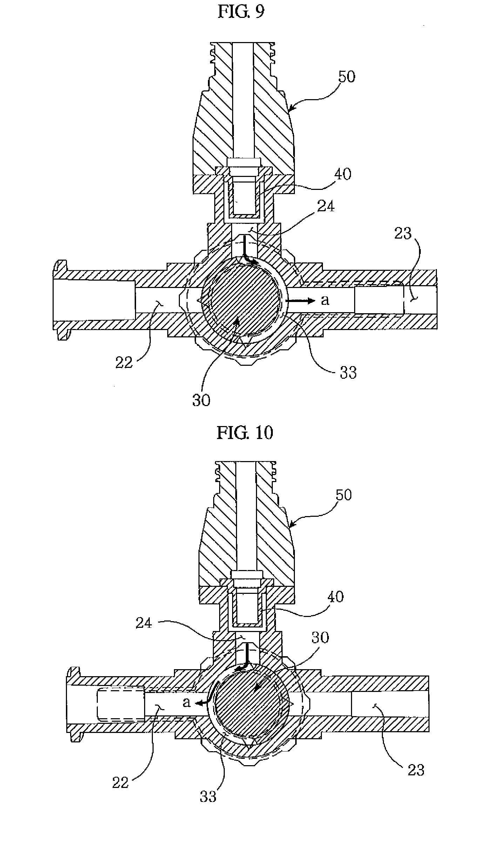

### (A) Preparation Step before Medicine is injected (see FIGS. 6 and 9)

Before charging the medicine injection apparatus 100 with a medicine, a cleaning medicinal fluid such as glucose or physiological saline is put into a syringe, and then the downstream conduit 152 is washed through the process shown in FIG. 6, so that a medicinal fluid is contained in a sponge (not shown) in the terminal cap 160.

First, the flow control unit 30 of the medicine charging and flow controlling device 10 according to the embodiment of the present invention is rotated by using the rotating handle 32 to be converted into the state of FIG. 9. In this case, as shown in FIG. 9, the connection passage 33 of the flow control unit 30 allows the medicine charging port 24 and the outlet port 23 to communicate with each other.

In such a state, if an external medicine supply device (for example, a syringe) is connected to the connection part 50 coupled to the valve body 20 and a medicinal fluid for cleaning syringe is injected thereto as shown in FIG. 6, the cleaning medicinal fluid is supplied along the arrow direction (direction "a") of FIG. 6 from the syringe through the downstream conduit 152 to the terminal cap 160. The medicinal fluid supplied through this path washes off impurities remaining in the downstream conduit 152 and makes the sponge in the terminal cap 160 sufficiently wet, thereby blocking the penetration of an external air.

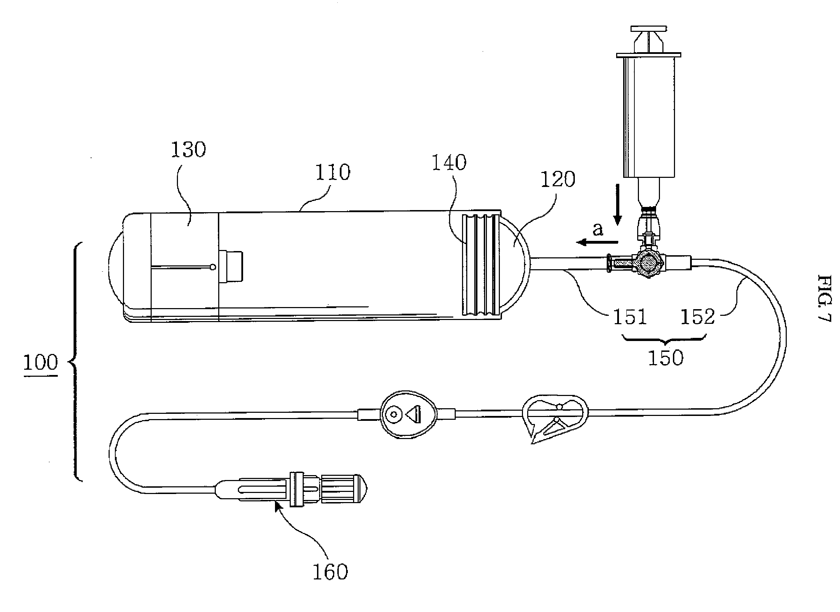

### (B) Medicine Charging Step (see FIGS. 7 and 10)

After the aforementioned preparation step, as shown in FIG. 10, the flow control unit 30 of the medicine charging and flow controlling device 10 according to the embodiment of the present invention is turned by using the rotating handle 32 so that the connection passage 33 of the flow control unit 30 may allow the medicine charging port 24 and the inlet port 22 to communicate with each other.

In this state, if the medicine of a medicine supply device such as a syringe is supplied by applying pressure to the medicine, the medicine opens the front outlet 42 of the one-way valve 40 disposed at the medicine charging port 24 by the applied pressure and passes through the front outlet 42, and flows along the arrow direction (direction "a") of FIG. 7 through the connection passage 33 of the flow control unit 30, the inlet port 22 and the upstream conduit 151 subsequently to be charged into the medicine storage space 120 of the cylinder 110.

During this process, even though the medicine charged in the medicine storage space 120 is pushed out toward the medicine charging port 24 by the pressure applied from the piston 140, the flow of the medicine is blocked by the one-way valve 40 installed to the medicine charging port 24, so that the medicine does not flow back toward the outside of the medicine charging port 24, namely toward the syringe.

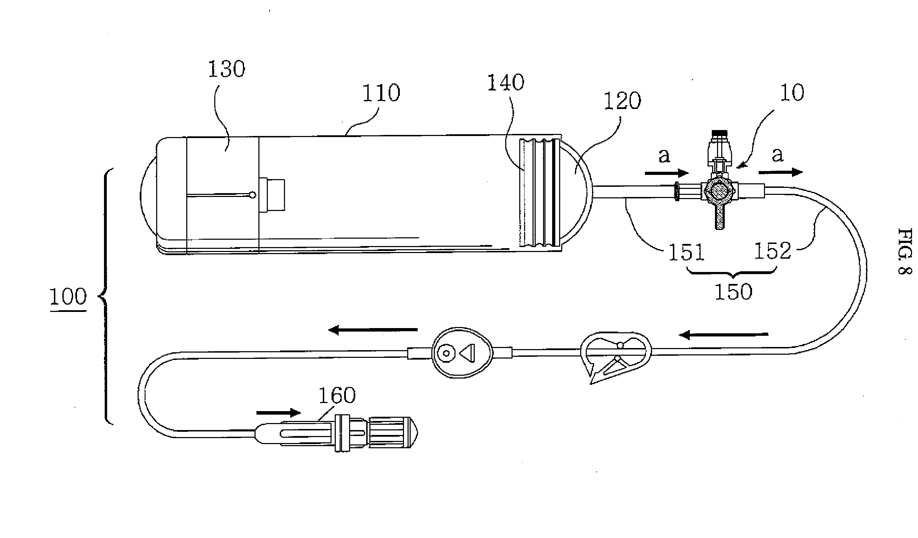

### (C) Medicine Injection Step (see FIGS. 8 and 11)

Once the medicine injection apparatus 100 is sufficiently charged with the medicine, the medicine supply device such as a syringe is separated from the connection part 50 in this state. In this case, since the outlet 42 of the one-way valve 40 installed to the medicine charging port 24 is closed, the introduction of an external air through the medicine charging port 24 is blocked, and simultaneously the medicine charged in the medicine storage space 120 is also blocked in order not to flow back to the outside through the medicine charging port 24.

Thereafter, if the flow control unit 30 is converted into the state shown in FIG 11 for the injection of the medicine, the connection passage 33 of the flow control unit 30 allows the inlet port 22 and the outlet port 23 to communicate with each other. In this state, the piston 140 of the medicine injection apparatus 100 is pressed by the gas discharged from the gas supply unit 130 to slowly move forward so that the medicine charged in the medicine storage space 120 is pushed out of the cylinder 110.

The medicine discharged from the cylinder 110 by the pressure of the piston 140 flows along the arrow direction (direction "a") of FIG. 8 through the upstream conduit 151, the inlet port 22 of the valve body 20, the connection passage 33 of the flow control unit 30, the outlet port 23 and the downstream conduit 152 subsequently, and is finally injected into a patient through a syringe needle or a catheter (not shown) connected to the terminal cap 160.

Even in this case, the medicine passing through the inlet port 22 and the connection passage 33 does not flow back out of the medicine charging port 24, and the air out of the medicine charging port 24 is also blocked in order not to be introduced.

Although preferred embodiments of the present invention have been described, the present invention is not limited thereto. It will be apparent that those skilled in the art can make various modifications and changes thereto without departing from the spirit and scope of the present invention and the modifications and changes are also included in the scope of the present invention.

## Claims

1. A medicine charging and flow controlling device, comprising:
a valve body having an inlet port communicating with an upstream conduit extending to communicate with a medicine storage space of a medicine injection apparatus, an outlet port communicating with a downstream conduit, and a medicine charging port allowing a medicine being introduced therein from the outside, the valve body having a hole in a central axis direction;
a flow control unit coupled with the valve body and having a connection passage allowing two of the inlet port, the outlet port and the medicine charging port to selectively communicate with each other, according to a rotation position thereof; and
a one-way valve installed to the medicine charging port of the valve body to allow the medicine to flow only in a direction from an inlet of the medicine charging port to an outlet thereof and to block the flow of the medicine in the opposite direction.

2. The medicine charging and flow controlling device as claimed in claim 1, wherein the flow control unit is rotatably coupled in the valve body and allows two ports among the inlet port, the outlet port and the medicine charging port to selectively communicate with each other.

3. The medicine charging and flow controlling device as claimed in claim 2, wherein the flow control unit is a swivel spool having a rotating handle.

4. The medicine charging and flow controlling device as claimed in claim 1, wherein the one-way valve is made of an elastic material and has an inlet and an outlet, and a cross section of a flowing path of the one-way valve gradually decreases from the inlet to the outlet so that the front end of the outlet is closed by both sidewalls being in close contact with each other by elasticity.

5. The medicine charging and flow controlling device as claimed in claim 4, wherein the one-way valve comprises a duckbill valve.

6. The medicine charging and flow controlling device as claimed in any one of claim 1 to 3, wherein the connection passage provided in the flow control unit is formed on the sidewall of the flow control unit along the circumferential direction, and the connection passage is formed in a range greater than 180 degrees and not more than 190 degrees with respect to the central axis.

7. The medicine charging and flow controlling device as claimed in claim 1, wherein a connection part for connecting an external medicine supply device for supplying a medicine from the outside is coupled to the medicine charging port of the valve body.

8. The medicine charging and flow controlling device as claimed in claim 7, wherein the connection part is a swivel connection part for rotatably connecting the external medicine supply device.

9. The medicine charging and flow controlling device as claimed in claim 7 or 8, wherein the connection part includes a medicine charging port coupling unit coupled to the medicine charging port, and a rotatable connection unit for rotatably connecting the external medicine supply device.

10. A medicine injection apparatus comprising the medicine charging and flow controlling device as claimed in any one of claim 1 to 5.
